# EUROPEAN PATENT APPLICATION

(11) **EP 4 620 485 A1**
(43) Date of publication of application: **24.09.2025**
(21) Application number: 23890689.5
(22) Date of filing: 10.11.2023
(51) Int. Cl.: A61K 47/36, A61K 9/16, C08J 3/12, C08L 5/08, A61K 31/728, A61K 47/04, A61P 19/02, A61P 17/02

(54) **HYALURONIC ACID PARTICLES AND PREPARATION METHOD THEREFOR**

(30) Priority: 17.11.2022 CN 202211459433
(71) Applicant: Bloomage Biotechnology Corporation Limited, Jinan, Shandong 250101 (CN); Bloomage Biotech (Tianjin) Co., Ltd., Tianjin 300270 (CN)
(72) Inventor: QIAN, Xiaolu, Jinan, Shandong 250101 (CN); WANG, Guanfeng, Jinan, Shandong 250101 (CN); ZHAO, Lianzhen, Jinan, Shandong 250101 (CN); DONG, Yanmei, Jinan, Shandong 250101 (CN); GUO, Xueping, Jinan, Shandong 250101 (CN)
(74) Representative: Abel & Imray LLP
(86) International application number: PCT/CN2023/130868
(87) International publication number: WO 2024/104255

(57) **Abstract**

The present application provides hyaluronic acid particles and a preparation method therefor. The preparation method comprises the following steps: carrying out airflow suspension on a hyaluronic acid raw material, and spraying an adhesive to the suspended hyaluronic acid raw material for granulation to obtain the hyaluronic acid particles, wherein the adhesive is an aqueous solution of water or hyaluronic acid. According to the preparation method of the present application, the dissolution rate of the HA can be significantly increased, the fluidity of the HA can also be improved, thereby avoiding caking, and reducing dust, waste and pollution, and it is more convenient to add the HA into a formula. When the preparation method of the present application is used for preparing the hyaluronic acid particles, three procedures of mixing, granulation and drying can be completed at a time in a container, such that the process route is simplified, the production period is shortened, the cost is reduced, dust is prevented from becoming airborne, and the material loss is reduced. In addition, according to the preparation method of the present application, during granulation, the temperature is relatively low, and the speed is fast, such that the preparation method is very suitable for the granulation of heat-sensitive substances such as hyaluronic acid.

## Description

### TECHNICAL FIELD

The present application belongs to the field of biomedicine, and specifically relates to hyaluronic acid particles and a preparation method thereof.

### BACKGROUND ART

Hyaluronic acid (HA) is a high-molecular-weight mucopolysaccharide composed of N-acetylglucosamine and D-glucuronic acid as structural units. Due to its unique molecular structure and physical and chemical properties, it exhibits a variety of important physiological functions in the human body, such as lubricating joints, regulating the permeability of blood vessel walls, regulating the diffusion and transport of proteins, water, and electrolytes, and promoting wound healing. Ordinary hyaluronic acid exists in powder form. When it dissolves in pure aqueous solution, the aggregation of powder and the formation of hydration film will seriously affect its dissolution rate and dissolution time. In particular, the higher the molecular weight of the hyaluronic acid, the more difficult it is to dissolve. Therefore, in order to improve the problem of difficult dissolution of hyaluronic acid and related products, it can be granulated. After granulation, its solubility can be improved, and its solid fluidity can also be improved.

In addition, different excipients can be selected and added during the granulation process, which can not only further increase the solubility, but also increase the diversity of efficacy. There are currently four commonly used granulation processes: wet extrusion/shear granulation, wet mixing granulation, dry granulation, and boiling granulation. The HA particles obtained by the first three granulation processes are compact particles with high bulk density and relatively hard texture. Although the problem of powder aggregation of HA particles can be solved to a certain extent during the dissolution in water, the formation of hydration film still cannot be overcome, so the degree of improving the dissolution rate is limited.

### CONTENTS OF THE APPLICATION

In view of the above problems existing in the prior art, the present application provides hyaluronic acid particles and a preparation method therefor.

Specifically, this application comprises the following aspects:
1. A method for preparing hyaluronic acid particles, comprising the following steps:
   suspending a hyaluronic acid raw material in an air flow, and
   spraying an adhesive onto the suspended hyaluronic acid raw material for granulation to obtain hyaluronic acid particles,
   wherein the adhesive is water or a hyaluronic acid aqueous solution.
2. The preparation method according to item 1, wherein the mass ratio of the hyaluronic acid raw material to the adhesive is (0.8-2):1 during the granulation process.
3. The preparation method according to item 1, wherein the induced draft temperature is 50°C -100°C, preferably 50°C -70°C, during the granulation process.
4. The preparation method according to item 1, wherein the induced draft frequency is 15 Hz-50 Hz, preferably 20 Hz-25 Hz, during the granulation process.
5. The preparation method according to item 1, wherein the water spraying rate is 50 rpm/min-150 rpm/min, preferably 80 rpm/min-120 rpm/min, during the granulation process.
6. The preparation method according to item 1, wherein the atomization pressure is 0.10 MPa-0.40 MPa, preferably 0.20 MPa-0.3 MPa, during the granulation process.
7. The preparation method according to item 1, wherein the volume ratio of the feeding amount to the capacity of the equipment for granulation is 20%-60% during the granulation process.
8. The preparation method according to item 1, wherein in the hyaluronic acid aqueous solution, the molecular weight of the hyaluronic acid is 200,000 Da-2,000,000 Da, preferably 1,000,000 Da-2,000,000 Da, more preferably, the content of hyaluronic acid is 0.01wt%-1wt%, and further preferably, the content of hyaluronic acid is 0.1wt%-0.5wt%.
9. The preparation method according to any one of items 1 to 8, further comprising sieving the hyaluronic acid particles.
10. A hyaluronic acid particle prepared by the preparation method according to any one of items 1 to 9.

The preparation method of the present application can significantly increase the dissolution rate of HA, improve its fluidity, prevent agglomeration, reduce dust waste and pollution, and is more convenient when added to the formula. The preparation method for preparing hyaluronic acid particles of the present application can complete the three steps of mixing, granulation and drying in a container at one time, thereby simplifying the process route, shortening the production cycle, reducing costs, preventing dust from flying, and reducing material loss. Moreover, the temperature in the granulation process of the preparation method of the present application is relatively low and the speed is fast, which is very suitable for the granulation of heat-sensitive substances such as hyaluronic acid.

### DETAILED DESCRIPTION

The present application is further described below in combination with examples. It should be understood that the examples are only used to further describe and illustrate the present application and are not intended to limit the present application.

Unless otherwise defined, technical and scientific terms used in this specification have the same meanings as commonly understood by those skilled in the art. Although methods and materials similar or equivalent to those described herein can be used in experiments or applications, the materials and methods are described below. In case of conflict, the present specification, including definitions, will control and, further, the materials, methods, and examples are illustrative only and not limiting. The present application is further described below in combination with specific examples, which is not intended to limit the scope of the present application.

The present application provides a method for preparing hyaluronic acid particles, comprising the following steps:
Step 1: suspending the hyaluronic acid raw material in an air flow.
Step 2: spraying an adhesive onto the suspended hyaluronic acid raw material for granulation to obtain hyaluronic acid particles, wherein the adhesive is water or a hyaluronic acid aqueous solution.

In step 1, the hyaluronic acid raw material is a hyaluronic acid powder raw material that has not been granulated, for example, can be any commercially available hyaluronic acid powder raw material, or hyaluronic acid powder directly obtained through a fermentation process.

Those skilled in the art will understand that in the present application, hyaluronic acid comprises hyaluronic acid and salts and derivatives thereof, including but not limited to sodium hyaluronate, potassium hyaluronate, zinc hyaluronate, calcium hyaluronate, magnesium hyaluronate, acetylated hyaluronic acid, thiolated hyaluronic acid, lightly cross-linked hyaluronic acid, etc.

The molecular weight of the hyaluronic acid raw material is not limited, and hyaluronic acid with various molecular weights is suitable for the method of the present application.

In a specific embodiment, the molecular weight of the hyaluronic acid raw material is 1000 Da-3,000,000 Da, for example, it may be 1000 Da, 10,000 Da, 50,000 Da, 100,000 Da, 200,000 Da, 300,000 Da, 400,000 Da, 500,000 Da, 600,000 Da, 700,000 Da, 800,000 Da, 900,000 Da, 1,000,000 Da, 1,100,000 Da, 1,200,000 Da, 1,300,000 Da, 1,400,000 Da, 1,500,000 Da, 1,600,000 Da, 1,700,000 Da, 1,800,000 Da, 1,900,000 Da, 2,000,000 Da, 2,100,000 Da, 2,200,000 Da, 2,300,000 Da, 2,400,000 Da, 2,500,000 Da, 2,600,000 Da, 2,700,000 Da, 2,800,000 Da, 2,900,000 Da, and 3,000,000 Da. Preferably, it may be 10,000 Da-2,000,000 Da, more preferably 200,000 Da-400,000 Da, 600,000 Da-1,000,000 Da, or 1,300,000 Da-1,600,000 Da.

The use of air flow suspension refers to suspending the sodium hyaluronate raw material with air in order to keep the sodium hyaluronate raw material in a fluidized state and to fully disperse it. Air flow suspension can be achieved using a boiling granulation apparatus or a fluidized bed granulation apparatus known in the art. In the present application, the air flow temperature for air flow suspension is called the induced draft temperature, that is, the air flow temperature at which the air flow enters the bottom of the fluidizing chamber and drives the material to suspend after being heated by the heating device. The higher the induced draft temperature used in air flow suspension, the shorter the granulation time and the less the water consumption, but the more severe the molecular weight degradation of HA molecules.

In a specific embodiment, the induced draft temperature for air flow suspension is 50°C-100°C, for example, it may be 50°C, 55°C, 60°C, 65°C, 70°C, 75°C, 80°C, 85°C, 90°C, or 100°C.

In this application, the induced draft frequency is a parameter that controls the air flow velocity, that is, the working frequency of the fan when extracting air flow. The higher the induced draft frequency for air flow suspension, the larger the granulation rate, but a high induced draft frequency will increase the breakage of the particles.

In a specific embodiment, the induced draft frequency for air flow suspension is 15 Hz-50 Hz, for example, it may be 15 Hz, 20 Hz, 25 Hz, 30 Hz, 35 Hz, 40 Hz, 45 Hz, or 50 Hz.

The feeding amount of hyaluronic acid raw material can be adjusted correspondingly according to the equipment for granulation. The higher the feeding amount, the longer the granulation time and the more water is used. If the feeding amount is too high, the boiling will be uneven, resulting in excessive extension of the granulation time. If the feeding amount is too low, the equipment utilization rate will be poor.

In a specific embodiment, the volume ratio of the feeding amount to the capacity of the equipment for granulation is 20%-60%, that is, the ratio of the feed volume to the equipment capacity is 20%-60%, for example, it may be 20%, 25%, 30%, 35%, 40%, 45%, 50%, 55%, or 60%.

In step 2, the adhesive may be sprayed in any manner that is conducive to uniform distribution of the adhesive.

In a specific embodiment, the adhesive is sprayed in the form of a mist-like aqueous solution.

The atomization pressure refers to the pressure of compressed air that causes the adhesive to be sprayed into a atomization state. The higher the atomization pressure, the shorter the granulation time. However, when the atomization pressure is too high, the spray angle of the adhesive after atomization will be too large and the adhesive will be sprayed onto the wall of the container, causing the material loss due to agglomerating on the wall.

In a specific embodiment, the adhesive is sprayed in the form of a mist-like aqueous solution. The atomization pressure is 0.1 MPa-0.4 MPa, for example, it may be 0.1 MPa, 0.15 MPa, 0.2 MPa, 0.25 MPa, 0.3 MPa, 0.35 MPa, or 0.4 MPa.

The greater the water spraying rate when the adhesive is sprayed, the larger the granulation rate is, but if the water spraying rate is too large, the particles are prone to agglomeration.

In a specific embodiment, the water spraying rate during adhesive spraying is 50 rpm/min-150 rpm/min, for example, it may be 50 rpm/min, 60 rpm/min, 70 rpm/min, 80 rpm/min, 90 rpm/min, 100 rpm/min, 110 rpm/min, 120 rpm/min, 130 rpm/min, 140 rpm/min, or 150 rpm/min.

When the adhesive used is a hyaluronic acid aqueous solution, the specific type and molecular weight of the hyaluronic acid used in the aqueous solution may be the same as or completely different from those of the hyaluronic acid raw material.

The breakage is related to the molecular weight of hyaluronic acid in the adhesive. Generally, the higher the molecular weight of hyaluronic acid in the adhesive, the smaller the breakage and the greater the hardness. The granulation time decreases with the increase of molecular weight of the adhesive.

In a specific embodiment, the molecular weight of hyaluronic acid in the hyaluronic acid aqueous solution is 200,000 Da-2,000,000 Da, for example, it may be 200,000 Da, 300,000 Da, 400,000 Da, 500,000 Da, 600,000 Da, 700,000 Da, 800,000 Da, 900,000 Da, 1,000,000 Da, 1,100,000 Da, 1,200,000 Da, 1,300,000 Da, 1,400,000 Da, 1,500,000 Da, 1,600,000 Da, 1,700,000 Da, 1,800,000 Da, 1,900,000 Da, or 2,000,000 Da.

In a specific embodiment, the content of hyaluronic acid in the hyaluronic acid aqueous solution is 0.01wt%-1wt%, for example, it may be 0.01wt%, 0.05wt%, 0.1wt%, 0.2wt%, 0.3wt%, 0.4wt%, 0.5wt%, 0.6wt%, 0.7wt%, 0.8wt%, 0.9wt%, or 0.1wt%.

In a specific embodiment, during the granulation process, the mass ratio of the hyaluronic acid raw material to the adhesive is (0.8-2):1, for example, it may be 0.8:1, 0.9:1, 1:1, 1.1:1, 1.2:1, 1.3:1, 1.4:1, 1.5:1, 1.6:1, 1.7:1, 1.8:1, or 2:1.

In a specific embodiment, the method for preparing hyaluronic acid particles of the present application comprises the following steps: Step 1: suspending the hyaluronic acid raw material in an air flow, wherein the volume ratio of the feeding amount to the capacity of the equipment for granulation is 20%-60%, the induced draft temperature is 50-100°C, and the induced draft frequency is 15 Hz-50 Hz. Step 2: spraying a hyaluronic acid aqueous solution onto the suspended hyaluronic acid raw material for granulation to obtain hyaluronic acid particles, wherein during the granulation process, the water spraying rate is 50 rpm/min-150 rpm/min, the atomization pressure is 0.10-0.40 MPa, the molecular weight of hyaluronic acid in the hyaluronic acid aqueous solution is 200,000 Da-2,000,000 Da, the content of hyaluronic acid is 0.01 wt%-1 wt%, and the mass ratio of the hyaluronic acid raw material to the hyaluronic acid aqueous solution is (0.8-2):1.

In a specific embodiment, the method for preparing hyaluronic acid particles of the present application comprises the following steps: Step 1: suspending the hyaluronic acid raw material in an air flow, wherein the volume ratio of the feeding amount to the capacity of the equipment for granulation is 20 %-60 %, the induced draft temperature is 50°C-70°C, and the induced draft frequency is 20 Hz-25 Hz. Step 2: spraying a hyaluronic acid aqueous solution onto the suspended hyaluronic acid raw material for granulation to obtain hyaluronic acid particles, wherein during the granulation process, the water spraying rate is 80 rpm/min-120 rpm/min, the atomization pressure is 0.20 MPa-0.3 MPa, the molecular weight of the hyaluronic acid in the hyaluronic acid aqueous solution is 1,000,000 Da-2,000,000 Da, the content of hyaluronic acid is 0.1 wt%-0.5 wt%, and the mass ratio of the hyaluronic acid raw material to the hyaluronic acid aqueous solution is (0.8-2):1.

In a specific embodiment, the method for preparing hyaluronic acid particles of the present application comprises the following steps: Step 1: suspending the hyaluronic acid raw material in an air flow, wherein the volume ratio of the feeding amount to the capacity of the equipment for granulation is 20%-60%, the induced draft temperature is 50°C-100°C, and the induced draft frequency is 15 Hz-50 Hz. Step 2: spraying water onto the suspended hyaluronic acid raw material for granulation to obtain hyaluronic acid particles, wherein during the granulation process, the water spraying rate is 50 rpm/min-150 rpm/min, the atomization pressure is 0.10-0.40 MPa, and the mass ratio of the hyaluronic acid raw material to water is (0.8-2):1.

In a specific embodiment, the method for preparing hyaluronic acid particles of the present application comprises the following steps: Step 1: suspending the hyaluronic acid raw material in an air flow, wherein the volume ratio of the feeding amount to the capacity of the equipment for granulation is 20%-60%, the induced draft temperature is 50°C-70°C, and the induced draft frequency is 20 Hz-25 Hz. Step 2: spraying water onto the suspended hyaluronic acid raw material for granulation to obtain hyaluronic acid particles, wherein during the granulation process, the water spraying rate is 80 rpm/min-120 rpm/min, the atomization pressure is 0.20-0.3M Pa, and the mass ratio of the hyaluronic acid raw material to water is (0.8-2):1.

Those skilled in the art will appreciate that step 1 and step 2 can be performed using any method and equipment known in the prior art.

In a specific embodiment, the preparation method consisting of step 1 and step 2 is a boiling granulation method. Boiling granulation is also called fluidized bed granulation or one-step granulation. It is a process of putting materials into a closed container at one time, mixing the materials evenly in the container, and then spraying the adhesive at a uniform speed through the equipment to allow the adhesive and materials to be fully mixed and flow in the container to form small particles, and then the hot air is sent in through the bottom to dry the wet particles, and finally the finished dry particles are directly collected.

The boiling granulation method can be implemented using an existing boiling granulation system, which usually comprises an air heating system, a spray system, a dust removal and screening system, etc. The principle is that the powdered material is suspended and circulated in a fluidized state due to the action of the hot air flow to achieve uniform mixing. At the same time, a mist-like adhesive is sprayed into the container to wet the powder, so that the powder is condensed into loose small particles. While granulating, the hot air flow efficiently dries the loose small particles, the water evaporates continuously, and the powder solidifies continuously. The process is repeated to form ideal, and uniform multi-microporous spherical particles. The hyaluronic acid particles obtained by this method are multi-microporous spherical particles, which increase the specific surface area and greatly increase the contact area with water during the dissolution process, overcome the formation of hydration film, and qualitatively improve the dissolution time.

In a specific embodiment, the method for preparing hyaluronic acid particles of the present application comprises the following steps: granulating the hyaluronic acid raw material using a boiling granulation method to obtain hyaluronic acid particles, wherein the adhesive used in the granulation process is a hyaluronic acid aqueous solution, and during the granulation process, the volume ratio of the feeding amount to the capacity of the equipment for granulation is 20%-60%, the induced draft temperature is 50°C-100°C, the induced draft frequency is 15 Hz-50 Hz, the water spraying rate is 50 rpm/min-150 rpm/min, the atomization pressure is 0.10 MPa-0.40 MPa, the molecular weight of hyaluronic acid in the hyaluronic acid aqueous solution is 200,000 Da-2,000,000 Da, the content of hyaluronic acid is 0.01 wt%-1 wt%, and the mass ratio of the hyaluronic acid raw material to the hyaluronic acid aqueous solution is (0.8-2):1.

In a specific embodiment, the method for preparing hyaluronic acid particles of the present application comprises the following steps: granulating the hyaluronic acid raw material using a boiling granulation method to obtain hyaluronic acid particles, wherein the adhesive used in the granulation process is a hyaluronic acid aqueous solution, and during the granulation process, the volume ratio of the feeding amount to the capacity of the equipment for granulation is 20%-60%, the induced draft temperature is 50°C-70°C, the induced draft frequency is 20 Hz-25 Hz, the water spraying rate is 80 rpm/min-120 rpm/min, the atomization pressure is 0.2 MPa-0.3 MPa, the molecular weight of hyaluronic acid in the hyaluronic acid aqueous solution is 1,000,000 Da-2,000,000 Da, the content of hyaluronic acid is 0.1wt%-0.5wt%, and the mass ratio of the hyaluronic acid raw material to the hyaluronic acid aqueous solution is (0.8-2):1.

In a specific embodiment, the method for preparing hyaluronic acid particles of the present application comprises the following steps: granulating the hyaluronic acid raw material using a boiling granulation method to obtain hyaluronic acid particles, wherein the adhesive used in the granulation process is water, and during the granulation process, the volume ratio of the feeding amount to the capacity of the equipment used for granulation is 20%-60%, the induced draft temperature is 50°C-100°C, the induced draft frequency is 15 Hz-50 Hz, the water spraying rate is 50 rpm/min-150rpm/min, the atomization pressure is 0.10 MPa-0.40MPa, and the mass ratio of the hyaluronic acid raw material to water is (0.8-2):1.

In a specific embodiment, the method for preparing hyaluronic acid particles of the present application comprises the following steps: granulating the hyaluronic acid raw material using a boiling granulation method to obtain hyaluronic acid particles, wherein the adhesive used in the granulation process is water, and during the granulation process, the volume ratio of the feeding amount to the capacity of the equipment for granulation is 20%-60%, the induced draft temperature is 50°C-70°C, the induced draft frequency is 20 Hz-25 Hz, the water spraying rate is 80 rpm/min-120 rpm/min, the atomization pressure is 0.2 MPa-0.3 MPa, and the mass ratio of the hyaluronic acid raw material to water is (0.8-2): 1.

The preparation method of the present application may further comprise step 3 after step 2:
Sieving the hyaluronic acid particles.

The purpose of sieving is to obtain hyaluronic acid particles with relatively uniform particle size. The specific size for sieving may be adjusted according to the needs of the actual hyaluronate product. For example, a 10-150 mesh sieve, a 40-60 mesh sieve, or a 60-120 mesh sieve may be used in the sieving step.

The present application also provides hyaluronic acid particles prepared by any one of the above preparation methods.

The preparation method of the present application shortens the granulation time, so that the hardness and solubility of the hyaluronic acid raw material are increased and the purity of the raw material is maintained in the prepared particles.

### Example

### Example 1

A certain amount of sodium hyaluronate raw material (the molecular weight of the sodium hyaluronate raw material is 520,000 Da) was prepared, and granulation of the sodium hyaluronate raw material was performed using an FL-120 boiling granulator (with 120 L of equipment capacity), and using purified water as an adhesive to obtain hyaluronic acid particles.

Among them, a total of 19 batches were prepared with different feeding amounts, induced draft temperatures, induced draft frequencies, water spraying rates, and atomization pressures.

Specifically, the preparation conditions of each batch are shown in Table 1. Taking batch 1 as an example, during the granulation process, the feeding amount was 10 kg, the induced draft temperature was 65°C, the induced draft frequency was 25 Hz, the water spraying rate was 100 rpm/min, the atomization pressure was 0.20 MPa, the amount of purified water was 11.5 L, the granulation time was 35 min, and the granulation amount was 50%; that is, the mass ratio of the prepared hyaluronic acid particles to the hyaluronic acid raw material was 50%.

**Table 1**

| batch | feeding amount (kg) | induced draft temperature | water spraying rate | induced draft frequency | atomization pressure | amount of purified water | granulation time | granulation amount |
|---|---|---|---|---|---|---|---|---|
| 1 | 10 | 65°C | 100rpm | 25Hz | 0.20MPa | 11.5L | 35min | 50% |
| 2 | 30 | 65°C | 100rpm | 25Hz | 0.20MPa | 16.5L | 50min | 50% |
| 3 | 40 | 65°C | 100rpm | 25Hz | 0.20MPa | 20L | 60min | 50% |
| 4 | *50* | 65°C | 100rpm | 25Hz | 0.20MPa | 25L | 75min | 50% |
| 5 | 60 | 65°C | 100rpm | 25Hz | 0.20MPa | 50L | 150min | 50% |
| 6 | 40 | 60°C | 100rpm | 25Hz | 0.20MPa | 25 L | 75 min | 50% |
| 7 | 40 | 70°C | 100rpm | 25Hz | 0.20MPa | 18 L | 55 min | 50% |
| 8 | 40 | 75°C | 100rpm | 25Hz | 0.20MPa | 13 L | 40 min | 50% |
| 9 | 40 | 80°C | 100rpm | 25Hz | 0.20MPa | 8 L | 25 min | 50% |
| 10 | 40 | 65°C | 50 rpm | 25Hz | 0.20MPa | 30 L | 90 min | 50% |
| 11 | 40 | 65°C | 80 rpm | 25Hz | 0.20MPa | 20 L | 65 min | 50% |
| 12 | 40 | 65°C | 120 rpm | 25Hz | 0.20MPa | 20 L | 55 min | 50% |
| 13 | 40 | 65°C | 150 rpm | 25Hz | 0.20MPa | 6.5 L | 20 min | 50% |
| 14 | 40 | 65°C | 100rpm | 15 Hz | 0.20MPa | 25 L | 75 min | 60% |
| 15 | 40 | 65°C | 100rpm | 20 Hz | 0.20MPa | 20 L | 65 min | 50% |
| 16 | 40 | 65°C | 100rpm | 30 Hz | 0.20MPa | 26.5 L | 80 min | 30% |
| 17 | 40 | 65°C | 100rpm | 25 Hz | 0.10MPa | 25 L | 75 min | 50% |
| 18 | 40 | 65°C | 100rpm | 25 Hz | 0.30MPa | 20 L | 55 min | 50% |
| 19 | 40 | 65°C | 100rpm | 25 Hz | 0.40MPa | 15 L | 45 min | 40% |

The molecular weight degradation rate, agglomeration rate and breakage rate of the hyaluronic acid particles prepared in each batch were measured respectively. The results are shown in Table 2.

Among them, the molecular weight degradation rate refers to the ratio of the molecular weight reduction to the original molecular weight measured by the intrinsic viscosity method.

The agglomeration rate refers to the ratio of the weight of large particles that do not pass through the 20-mesh molecular sieve to the feeding amount.

The dissolution time is determined by the time taken to dissolve 0.4% of the particles in 100 mL of purified water.

The breakage rate refers to the mass ratio of broken particles to the original particles after vibration.

**Table 2**

| batch | molecular weight degradation rate | agglomeration rate | breakage rate | dissolution time |
|---|---|---|---|---|
| 1 | 8% | 0% | 50% | 2min (post-granulation) |
| 2 | 8% | 0% | 50% | |
| 3 | 8% | 0% | 50% | 20min (pre-granulation) |
| 4 | 8% | 0% | 50% | |
| 5 | 8% | 0% | 50% | |
| 6 | 5% | 0% | 50% | |
| 7 | 8% | 0% | 50% | |
| 8 | 12% | 0% | 50% | |
| 9 | 20% | 0% | 50% | |
| 10 | 8% | 0% | 50% | |
| 11 | 8% | 0% | 50% | |
| 12 | 8% | 0% | 50% | |
| 13 | 8% | 5% | 50% | |
| 14 | 8% | 0% | 40% | |
| 15 | 8% | 0% | 50% | |
| 16 | 8% | 0% | 70% | |
| 17 | 8% | 0% | 50% | |
| 18 | 8% | 0% | 50% | |
| 19 | 8% | 0% | 50% | |

It can be seen from the results in Table 2 that for batches 1-5, under the same conditions of induced draft temperature, water spraying rate, fan frequency, atomization pressure and granulation rate, the higher the feeding amount, the longer the granulation time and the more water used. If the feeding amount is too high, the boiling will be uneven, resulting in excessive extension of the granulation time. If the feeding amount is too low, the equipment utilization rate will be poor. Therefore, the optimal feeding amount is 30-50kg after considering comprehensively.

By comparing batches 3 and 6-9, it can be seen that under the same conditions of feeding amount, water spraying rate, fan frequency, atomization pressure and granulation rate, the higher the induced draft temperature, the shorter the granulation time and the less water used, but the more severe the molecular weight degradation of the HA molecule. Comprehensively considering the factors of molecular weight degradation and granulation efficiency, it can be seen that the optimal induced draft temperature is 65-70°C.

By comparing batches 3 and 10-13, it can be seen that under the same conditions of feeding amount, induced draft temperature, fan frequency, atomization pressure and granulation rate, the greater the water spraying rate, the larger the granulation rate, but the particles are prone to agglomeration when exceeding 120 rpm/min. Therefore, the optimal water spraying rate is 80 rpm/min-120 rpm/min.

By comparing batches 3 and 14-16, it can be seen that under the same conditions of feeding amount, induced draft temperature, water spraying rate, atomization pressure and granulation rate, the higher the fan frequency, the larger the granulation rate. However, during drying, a high fan frequency will increase the breakage of the particles. Therefore, the optimal fan frequency is 20 Hz-25 Hz after considering comprehensively.

By comparing batches 3 and 17-19, it can be seen that under the same conditions of feeding amount, induced draft temperature, water spraying rate, fan frequency and granulation rate, the higher the atomization pressure, the shorter the granulation time. However, when the atomization pressure is too high, the spray angle of the adhesive after atomization will be too large and the adhesive will be sprayed onto the wall of the container, causing the material loss due to agglomerating on the wall. Therefore, the optimal atomization pressure is 0.20 MPa-0.3 MPa.

### Example 2

A certain amount of sodium hyaluronate raw material (the molecular weight of the sodium hyaluronate raw material is 520,000 Da or 1,230,000 Da) was prepared, granulation of the sodium hyaluronate raw material was performed using an FL-120 boiling granulator, and using purified water or an aqueous solution of sodium hyaluronate as an adhesive respectively to obtain hyaluronic acid particles.

Among them, four batches were prepared using hyaluronic acid raw materials with different molecular weights and different adhesives. During the preparation of these four batches, the feeding amount, induced draft temperature, induced draft frequency, water spraying rate, and atomization pressure were the same with each other. During the granulation process, the feeding amount was 40 kg, the induced draft temperature was 65°C, the induced draft frequency was 25 Hz, the water spraying rate was 100 rpm/min, and the atomization pressure was 0.20 MPa.

Specifically, the preparation conditions of each batch are shown in Table 3. Taking batch 1 as an example, the raw material used in the granulation process was sodium hyaluronate with a molecular weight of 520,000 Da, and the adhesive was a sodium hyaluronate aqueous solution with a mass concentration of 0.1% (the molecular weight of sodium hyaluronate in the solution is 520,000 Da). The granulation time was 30min, the amount of adhesive used was 8 L, and the granulation amount was 70%.

The particle size, dissolution time and granulation hardness of the hyaluronic acid particles prepared in each batch were measured respectively. The results are shown in Table 3.

Among them, the particle size refers to the mass ratio of the amount of particles passing through the sieve with corresponding mesh size to the total amount of particles. The breakage rate refers to the mass ratio of broken particles to the original particles after vibration.

**Table 3**

| batch | molecular weight of raw material HA | adhesive | granulation time | amount of adhesive used | granulation amount | particle size | dissolution time | breakage rate |
|---|---|---|---|---|---|---|---|---|
| 1 | 520,000 Da | 0.1% 520,000 Da HA | 30min | 8L | 70% | 70% > 60 mesh | 2min (post-granulation) | vibrating for 30min under 25 Hz breakage rate is 20% |
| 2 | 520,000 Da | purified water | 60min | 20L | 50% | 50% > 60 mesh | 20min (pre-granulation) | vibrating for 30min under 25 Hz breakage rate is 60% |
| 3 | 1,230,000 Da | 0.1% 520,000 Da HA | 25min | 8L | 60% | 50% > 60 mesh | 4min (post-granulation) | vibrating for 30min under 25 Hz breakage rate is 30% |
| 4 | 1,230,000 Da | purified water | 50min | 18L | 30% | 30% > 60 mesh | 30min (pre-granulation) | vibrating for 30min under 25 Hz breakage rate is 70% |

From the results in Table 3, it can be seen that when the aqueous solution of hyaluronic acid, which is the same as the raw material, is used as an adhesive for granulation, the granulation time is short, the hardness is high, less water is used for granulation, the solubility remains unchanged, other substances will not be introduced, and the purity of the product itself can be maintained.

### Example 3

A certain amount of sodium hyaluronate raw material (the molecular weight of the sodium hyaluronate raw material is 520,000 Da, 1,230,000 Da or 1,710,000 Da) was prepared, granulation of the sodium hyaluronate raw material was performed using an FL-120 boiling granulator, and suing a sodium hyaluronate aqueous solution with a mass concentration of 1% as an adhesive to obtain hyaluronic acid particles.

Among them, 9 batches were prepared using hyaluronic acid raw materials and adhesives with different molecular weights. During the preparation of these 9 batches, the feeding amount, induced draft temperature, induced draft frequency, water spraying rate, and atomization pressure were the same with each other. During the granulation process, the feeding amount was 40 kg, the induced draft temperature was 65°C, the induced draft frequency was 25 Hz, the water spraying rate was 100rpm/min, and the atomization pressure was 0.20 MPa.

Specifically, the preparation conditions of each batch are shown in Table 4. Taking batch 1 as an example, the raw material used in the granulation process was sodium hyaluronate with a molecular weight of 520,000 Da, and the adhesive was a sodium hyaluronate aqueous solution with a mass concentration of 0.1% (the molecular weight of sodium hyaluronate in the solution is 520,000 Da). The granulation time was 30min, the amount of adhesive used was 8 L, and the granulation amount was 70%.

The particle size, dissolution time and granulation hardness (i.e., the breakage rate) of the hyaluronic acid particles prepared in each batch were measured respectively. The results are shown in Table 4.

**Table 4**

| batch | molecular weight of raw material HA | adhesive | granulation time | amount of adhesive used | granulation amount | particle size | dissolution time | breakage rate |
|---|---|---|---|---|---|---|---|---|
| 1 | 520,000 Da | 0.1% 520,000 Da HA | 30min | 8L | 70% | 70% > 60 mesh | 2min(post-granulation) 20min(pre-granulation) | vibrating for 30min under 25Hz breakage rate is 20% |
| 2 | 520,000 Da | 0.1% 1,230,000 Da HA | 20min | 7L | 70% | 70% > 60 mesh | | vibrating for 30min under 25Hz breakage rate is 15% |
| 3 | 520,000 Da | 0.1% 1,710,000 Da HA | 18min | 5L | 70% | 70% > 60 mesh | | vibrating for 30min under 25Hz breakage rate is 15% |
| 4 | 1,230,000 Da | 0.1% 520,000 Da HA | 25min | 8L | 60% | 50% > 60 mesh | 4min(post-granulation) 30min(pre-granulation) | vibrating for 30min under 25Hz breakage rate is 30% |
| 5 | 1,230,000 Da | 0.1% 1,230,000 Da HA | 18min | 7L | 60% | *50%* > 60 mesh | | vibrating for 30min under 25 Hz breakage rate is 25% |
| 6 | 1,230,000 Da | 0.1% 1,710,000 Da HA | 15min | 6.5L | 60% | *50%* > 60 mesh | | vibrating for 30min under 25 Hz breakage rate is 25% |
| 7 | 1,710,000 Da | 0.1% 520,000 Da HA | 20min | 8L | 55% | 40% > 60 mesh | 4min(post-granulation) 40min(pre-granulation) | vibrating for 30min under 25 Hz breakage rate is 35% |
| 8 | 1,710,000 Da | 0.1% 1,230,000 Da HA | 16min | 7.5L | 55% | 40% > 60 mesh | | vibrating for 30min under 25 Hz breakage rate is 30% |
| 9 | 1,710,000 Da | 0.1% 1,710,000 Da HA | 15min | 6L | 55% | 40% > 60 mesh | | vibrating for 30min under 25 Hz breakage rate is 30% |

From the results in Table 4, it can be seen that using aqueous solutions of hyaluronic acid with different molecular weights as adhesives to granulate particles of hyaluronic acid raw materials with different molecular weights, pre- and post-granulation:
The dissolution time of hyaluronic acid raw materials with the same molecular weight changes consistently pre- and post-granulation, and has no obvious relationship with the molecular weight of hyaluronic acid in the selected adhesive. However, as the molecular weight of the hyaluronic acid raw materials increases, the dissolution time of its particles becomes longer, wherein for the dissolution time, particles of HA with 520,000 molecular weight < particles of HA with 1,230,000 molecular weight= particles of HA with 1,710,000 molecular weight.

The particle size of hyaluronic acid raw materials with the same molecular weight is consistent, and has no obvious relationship with the molecular weight in the selected adhesive, but the particle size will decrease with the increase of the molecular weight of the raw material.

The breakage is related to the molecular weight of the adhesive. The higher the molecular weight of hyaluronic acid in the adhesive, the smaller the breakage and the greater the hardness. However, the hardness of the particles prepared by adhesives with with a molecular weight of 1,230,000 Da and 1,710,000 Da is similar. Therefore, when the molecular weight of hyaluronic acid in the adhesive reaches a certain value, it has almost no effect on the hardness.

The granulation time decreases with the increase of the molecular weight of hyaluronic acid in the adhesive. The larger the granulation rate, the less adhesive is used.

Therefore, hyaluronic acid with a molecular weight of 1,000,000 Da,-2,000,000 Da has a better effect as an adhesive.

### Example 4

A certain amount of sodium hyaluronate raw material (the molecular weight of the sodium hyaluronate raw material is 520,000 Da) was prepared, granulation of the sodium hyaluronate raw material was performed using an FL-120 boiling granulator, and using sodium hyaluronate aqueous solutions with different mass concentrations as adhesives to obtain hyaluronic acid particles.

Among them, 5 batches were prepared using hyaluronic acid aqueous solutions with different concentrations as adhesives. During the preparation of these 5 batches, the feeding amount, induced draft temperature, induced draft frequency, water spraying rate, and atomization pressure were the same with each other. During the granulation process, the feeding amount was 40 kg, the induced draft temperature was 65°C, the induced draft frequency was 25 Hz, the water spraying rate was 100 rpm/min, and the atomization pressure was 0.20 MPa.

Specifically, the preparation conditions of each batch are shown in Table 5. Taking batch 1 as an example, the adhesive used in the granulation process was a sodium hyaluronate aqueous solution with a mass concentration of 0.1% (the molecular weight of sodium hyaluronate in the solution is 1,230,000 Da). The granulation time was 40min, the amount of adhesive used was 18L, and the granulation amount was 70%.

The particle size, dissolution time and granulation hardness (i.e., the breakage rate) of the hyaluronic acid particles prepared in each batch were measured respectively. The results are shown in Table 5.

**Table 5**

| batch | molecular weight of raw material HA | adhesive | granulation time | amount of adhesive used | granulation amount | particle size | dissolution time | breakage rate |
|---|---|---|---|---|---|---|---|---|
| 1 | 520,000 Da | 0.01% 1,230,000 Da HA | 40min | 18L | 70% | 60% > 60 mesh | 2min(post-granulation) 20min(pre-granulation) | vibrating for 30min under 25HZ breakage rate is 20% |
| 2 | 520,000 Da | 0.05% 1,230,000 Da HA | 30min | 12L | 70% | 70% > 60 mesh | | vibrating for 30min under 25HZ breakage rate is 16% |
| 3 | 520,000 Da | 0.1% 1,230,000 Da HA | 20min | 7L | 70% | 70% > 60 mesh | | vibrating for 30min under 25HZ breakage rate is 15% |
| 4 | 520,000 Da | 0.2% 1,230,000 Da HA | 18min | 6L | 70% | 75% > 60 mesh | | vibrating for 30min under 25HZ breakage rate is 15% |
| 5 | 520,000 Da | 0.5% 1,230,000 Da HA | 16min | 5L | 70% | 75% > 60 mesh | | vibrating for 30min under 25HZ breakage rate is 14% |

It can be seen from the data in Table 5 that when granulating hyaluronic acid raw materials with the same molecular weight with different concentrations of adhesives, the granulation time decreases with the increase of the adhesive concentration, and the change is not obvious when the mass concentration is higher than 0.1%; the amount also decreases with the increase of concentration, and the trend slows down when the mass concentration is higher than 0.1%; the granulation amount is independent of the concentration; the particle size increases with the increase of concentration; and the hardness becomes stronger, and there is no obvious change after it is higher than 0.1%. Based on the above data, it can be concluded that the mass concentration of 0.1%-0.5% is the preferred concentration.

## Claims

1. A method for preparing hyaluronic acid particles, comprising the following steps:
suspending a hyaluronic acid raw material in an air flow, and
spraying an adhesive onto the suspended hyaluronic acid raw material for granulation to obtain hyaluronic acid particles,
wherein the adhesive is water or a hyaluronic acid aqueous solution.

2. The method according to claim 1, wherein the mass ratio of the hyaluronic acid raw material to the adhesive is (0.8-2):1 during the granulation process.

3. The method according to claim 1, wherein the induced draft temperature is 50°C -100°C, preferably 50°C -70°C, during the granulation process.

4. The method according to claim 1, wherein the induced draft frequency is 15 Hz -50 Hz, preferably 20 Hz -25 Hz, during the granulation process.

5. The method according to claim 1, wherein the water spraying rate is 50 rpm/min-150 rpm/min, preferably 80 rpm/min-120 rpm/min, during the granulation process.

6. The method according to claim 1, wherein the atomization pressure is 0.10 MPa-0.40 MPa, preferably 0.20 MPa-0.3 MPa, during the granulation process.

7. The method according to claim 1, wherein the volume ratio of the feeding amount to the capacity of the equipment for granulation is 20%-60% during the granulation process.

8. The method according to claim 1, wherein, in the hyaluronic acid aqueous solution, the molecular weight of the hyaluronic acid is 200,000 Da-2,000,000 Da, preferably 1,000,000 Da-2,000,000 Da, more preferably, the content of hyaluronic acid is 0.01wt%-1wt%, and further preferably, the content of hyaluronic acid is 0.1wt%-0.5wt%.

9. The method according to any one of claims 1 to 8, further comprising sieving the hyaluronic acid particles.

10. A hyaluronic acid particle prepared by the method according to any one of claims 1 to 9.
